# EUROPEAN PATENT APPLICATION

(11) **EP 4 177 605 A1**
(43) Date of publication of application: **10.05.2023**
(21) Application number: 21833599.0
(22) Date of filing: 25.05.2021
(51) Int. Cl.: G01N 33/48, G01N 33/483, G01N 21/27

(54) **PATHOLOGICAL DIAGNOSIS ASSIST DEVICE, OPERATION METHOD OF PATHOLOGICAL DIAGNOSIS ASSIST DEVICE, AND OPERATION PROGRAM OF PATHOLOGICAL DIAGNOSIS ASSIST DEVICE**

(30) Priority: 02.07.2020 JP 2020115065
(71) Applicant: FUJIFILM Corporation, Tokyo 106-8620 (JP)
(72) Inventor: TOMINAGA, Shunsuke, Ashigarakami-gun, Kanagawa 258-8577 (JP)
(74) Representative: Klunker IP Patentanwälte PartG mbB
(86) International application number: PCT/JP2021/019793
(87) International publication number: WO 2022/004198

(57) **Abstract**

Provided are a pathological diagnosis support apparatus, an operation method for a pathological diagnosis support apparatus, and an operation program for a pathological diagnosis support apparatus, by which the detection accuracy of a fibrotic region can be improved. An RW control unit of the pathological diagnosis support apparatus acquires a sample image obtained by imaging a sample of a liver stained with Sirius red by reading it out from a storage device. A detection unit detects a fibrotic region of the liver by comparing, with a preset threshold value, a ratio between the pixel values of a G channel and an R channel among three color channels of RGB of the sample image. A derivation unit derives evaluation information indicating a degree of fibrosis of the liver based on the detected fibrotic region. A display control unit outputs the evaluation information by displaying an analysis result display screen including the evaluation information on a display.

## Description

### Technical Field

The technology of the present disclosure relates to a pathological diagnosis support apparatus, an operation method for a pathological diagnosis support apparatus, and an operation program for a pathological diagnosis support apparatus.

### Related Art

In pathological diagnosis, the degree of fibrosis of a biological tissue leading to a risk of a disease such as cancer is evaluated. Conventionally, a pathologist observes a sample collected from the biological tissue under a microscope and visually evaluates the degree of fibrosis. However, such an evaluation method imposes a heavy burden on the pathologist. Furthermore, the evaluation may vary depending on the skill level of the pathologist.

Therefore, for example, as in Vincenza Calvaruso et al., "Computer-Assisted Image Analysis of Liver Collagen: Relationship to Ishak Scoring and Hepatic Venous Pressure Gradient", published in April 2009, HEPATOLOGY (49), pp. 1236-1244, a method has been proposed in which an image of a sample (hereinafter referred to as a sample image) is analyzed by a computer to detect a fibrotic region, and the areal percentage of the fibrotic region or the like is derived and presented to a user such as a pathologist. Vincenza Calvaruso et al., "Computer-Assisted Image Analysis of Liver Collagen: Relationship to Ishak Scoring and Hepatic Venous Pressure Gradient", published in April 2009, HEPATOLOGY (49), pp. 1236-1244 uses a sample image of a sample in which a fibrotic region is stained with a dye such as Sirius red. Then, the fibrotic region is detected by comparing pixel values of color channels of RGB (Red, Green, and Blue) of the sample image with a threshold value that is preset by a user.

### SUMMARY OF THE INVENTION

### Technical Problem

The fibrotic region is not uniformly stained with the same color by staining with a dye such as Sirius red. For example, the color changes depending on the concentration of the dye or the ambient environment such as temperature and humidity. In addition, the color also changes depending on the difference in the imaging apparatus of the sample image. Therefore, as disclosed in Vincenza Calvaruso et al., "Computer-Assisted Image Analysis of Liver Collagen: Relationship to Ishak Scoring and Hepatic Venous Pressure Gradient", published in April 2009, HEPATOLOGY (49), pp. 1236-1244, a method for detecting a fibrotic region by simply comparing the pixel values of RGB color channels of the sample image with the threshold value has low detection accuracy of the fibrotic region.

An object of the technology of the present disclosure is to provide a pathological diagnosis support apparatus, an operation method for a pathological diagnosis support apparatus, and an operation program for a pathological diagnosis support apparatus, by which the detection accuracy of the fibrotic region can be improved.

### Solution to Problem

In order to achieve the above object, a pathological diagnosis support apparatus according to the present disclosure includes at least one processor, in which the processor is configured to: acquire a sample image obtained by imaging a sample of a biological tissue stained with a dye, the sample image having pixel values of a plurality of color channels; detect a fibrotic region of the biological tissue by comparing a ratio between pixel values of two color channels among the plurality of color channels with a preset threshold value; derive evaluation information indicating a degree of fibrosis of the biological tissue based on the detected fibrotic region; and output the evaluation information.

The processor preferably calculates a ratio between pixel values of two color channels corresponding to two color regions having a relatively large difference in absorbance in an absorption spectrum of the dye.

The processor preferably derives the evaluation information separately for a perivascular region and a non-perivascular region other than the perivascular region, the perivascular region including a periphery of a blood vessel passing through the biological tissue.

The sample is collected from a liver, and the processor derives the evaluation information separately for a pericentral region and the non-perivascular region, the pericentral region including a periphery of a central vein passing through the liver.

The processor preferably derives a numerical value related to at least one of an area or a length of the fibrotic region as the evaluation information.

The processor preferably detects the fibrotic region after performing compression processing on the acquired sample image.

In an operation method for a pathological diagnosis support apparatus according to the present disclosure, a processor executes: an acquisition process of acquiring a sample image obtained by imaging a sample of a biological tissue stained with a dye, the sample image having pixel values of a plurality of color channels; a detection process of detecting a fibrotic region of the biological tissue by comparing a ratio between pixel values of two color channels among the plurality of color channels with a preset threshold value; a derivation process of deriving evaluation information indicating a degree of fibrosis of the biological tissue based on the detected fibrotic region; and an output process of outputting the evaluation information.

In an operation program for a pathological diagnosis support apparatus according to the present disclosure, the program causes a processor to execute: an acquisition process of acquiring a sample image obtained by imaging a sample of a biological tissue stained with a dye, the sample image having pixel values of a plurality of color channels; a detection process of detecting a fibrotic region of the biological tissue by comparing a ratio between pixel values of two color channels among the plurality of color channels with a preset threshold value; a derivation process of deriving evaluation information indicating a degree of fibrosis of the biological tissue based on the detected fibrotic region; and an output process of outputting the evaluation information.

### Effects of Invention

According to the technology of the present disclosure, it is possible to provide a pathological diagnosis support apparatus, an operation method for a pathological diagnosis support apparatus, and an operation program for a pathological diagnosis support apparatus, by which the detection accuracy of the fibrotic region can be improved.

### BRIEF DESCRIPTION OF THE DRAWINGS

Fig. 1 is a diagram illustrating a pathological diagnosis process, a sample image, and a pathological diagnosis support apparatus;
Fig. 2 is a block diagram illustrating a computer constituting the pathological diagnosis support apparatus;
Fig. 3 is a block diagram illustrating a processing unit of a central processing unit (CPU) of the pathological diagnosis support apparatus;
Fig. 4 is a diagram illustrating processing performed by a detection unit according to an absorption spectrum of Sirius red;
Fig. 5 is a diagram illustrating processing performed by the detection unit in a case of staining with Sirius red;
Fig. 6 is a diagram illustrating detection results;
Fig. 7 is a diagram illustrating evaluation information;
Fig. 8 is a flowchart illustrating a procedure for deriving an areal percentage of a fibrotic region;
Fig. 9 is a diagram illustrating the procedure for deriving the areal percentage of the fibrotic region;
Fig. 10 is a flowchart illustrating a procedure for deriving a length percentage of a fibrotic region;
Fig. 11 is a diagram illustrating the procedure for deriving the length percentage of the fibrotic region;
Fig. 12 is a diagram illustrating an analysis result display screen;
Fig. 13 is a flowchart illustrating a processing procedure of the pathological diagnosis support apparatus;
Fig. 14 is a diagram illustrating processing performed by the detection unit according to an absorption spectrum of a dye used for Masson's trichrome staining;
Fig. 15 is a diagram illustrating processing performed by the detection unit in a case of Masson's trichrome staining;
Fig. 16 is a diagram illustrating a table in which levels representing degrees of fibrosis corresponding to ranges of the areal percentage of the fibrotic region are registered;
Fig. 17 is a diagram illustrating another example of the analysis result display screen;
Fig. 18 is a diagram illustrating another example of the analysis result display screen;
Fig. 19 is a diagram illustrating a processing unit according to a second embodiment;
Fig. 20 is a diagram illustrating a state in which a perivascular region and a non-perivascular region are extracted by a machine learning model;
Fig. 21 is a diagram illustrating an analysis result display screen according to the second embodiment;
Fig. 22 is an enlarged view of a sample image displayed on the analysis result display screen illustrated in Fig. 21;
Fig. 23 is a diagram illustrating another example of the analysis result display screen according to the second embodiment;
Fig. 24 is an enlarged view of a sample image displayed on the analysis result display screen illustrated in Fig. 23;
Fig. 25 is a diagram illustrating a processing unit according to a third embodiment;
Fig. 26 is a diagram illustrating a state in which a pericentral region, a periportal region, and a non-perivascular region are extracted by a machine learning model;
Fig. 27 is a diagram illustrating an analysis result display screen according to the third embodiment;
Fig. 28 is an enlarged view of a sample image displayed on the analysis result display screen illustrated in Fig. 27;
Figs. 29A and 29B are diagrams illustrating other examples of evaluation information according to the third embodiment, in which Fig. 29A illustrates evaluation information including an areal percentage and a length percentage of a fibrotic region in a periportal region, and Fig. 29B illustrates evaluation information including the number of P-P bridges and the number of P-C bridges;
Fig. 30 is a diagram illustrating a processing unit according to a fourth embodiment; and
Fig. 31 illustrates a sample collected from a lung of a patient.

### DESCRIPTION OF THE EMBODIMENTS

### First Embodiment

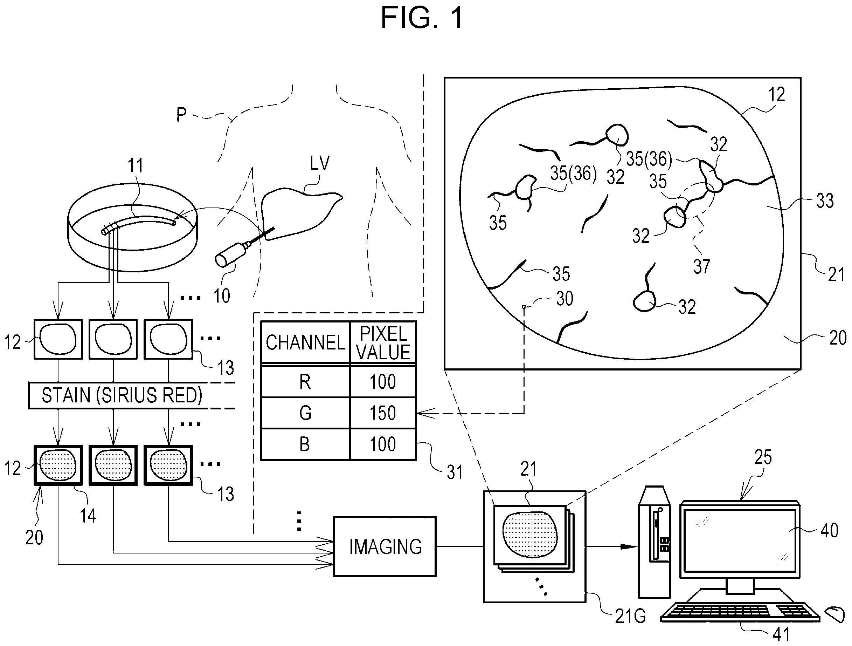

In Fig. 1, pathological diagnosis is performed, for example, through the following steps. First, a biopsy needle 10 is inserted into a liver LV from the outside of a body of a patient P under monitoring by an ultrasonic observation apparatus (omitted from illustration), and an elongated test substance 11 is collected from the liver LV. The test substance 11 is embedded in paraffin, and then sliced into a plurality of slices 12 by a microtome, and the sliced slices 12 are attached to pieces of glass 13. Subsequently, the paraffin is removed, the slices 12 are stained with a dye, and the stained slices 12 are covered with cover glasses 14 to complete samples 20. In the present example, Sirius red is used as the dye. The liver LV is an example of a "biological tissue" according to the technology of the present disclosure.

The samples 20 are each set in an imaging apparatus (omitted from illustration) such as a digital optical microscope and imaged. Sample images 21 of the samples 20 thus obtained are input to a pathological diagnosis support apparatus 25 as a sample image group 21G. A patient identification data (ID) for uniquely identifying the patient P, imaging date and time, and the like are attached to the sample images 21.

As illustrated in Table 31, the pixel values of the three color channels of RGB are assigned to pixels 30 of each sample image 21. Therefore, the sample image 21 is a full-color image. The pixel value is, for example, a numerical value in a range of 0 to 255.

The sample 20 including the slice 12 is captured in the sample image 21. The slice 12 has a vascular region 32 and a parenchymal region 33. The vascular region 32 is a region of a blood vessel passing through the liver LV, such as a portal vein and a central vein. The vascular region 32 is hollow in the slice 12. The parenchymal region 33 is a region where hepatocytes of the liver LV are present.

Sirius red stains a fibrotic region 35 (more specifically, the region of collagen fibers) red. In the sample image 21 illustrated in Fig. 1, fibrotic regions 35 are present in perivascular regions 36, which are regions around vascular regions 32, and in places in the parenchymal region 33. In addition, as surrounded by a two dot chain line frame 37, a fibrotic region 35 connecting two blood vessels, that is, bridging fibrosis, is also observed. Bridging fibrosis is referred to as P-P bridging when the two blood vessels are both in a portal vein region, or P-C bridging when the two blood vessels are a portal vein and a central vein.

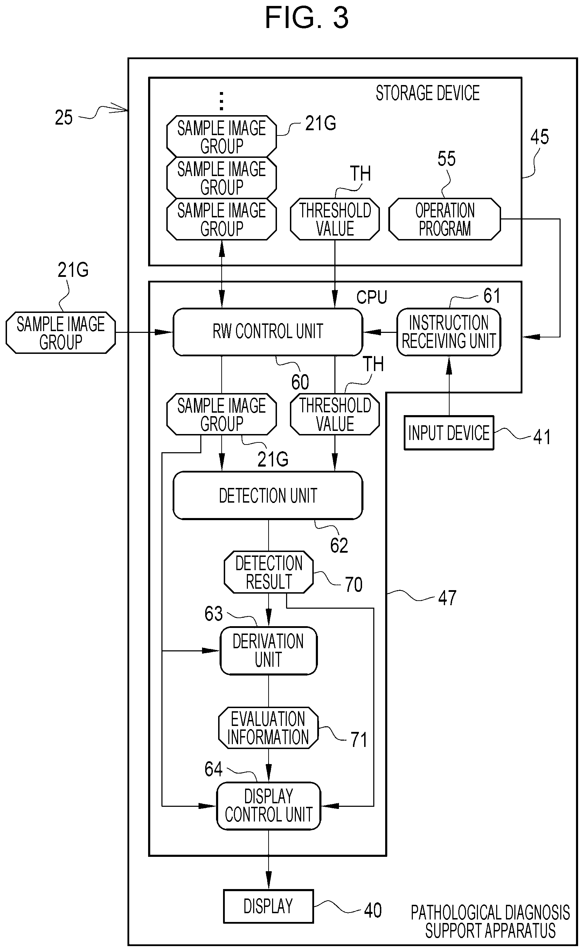

The pathological diagnosis support apparatus 25 is, for example, a desktop personal computer, and has a display 40 and an input device 41. The input device 41 is a keyboard, a mouse, a touch panel, or the like. The pathological diagnosis support apparatus 25 analyzes the sample image 21 to detect the fibrotic region 35. Then, the pathological diagnosis support apparatus 25 derives evaluation information 71 (see Fig. 3 and the like) such as the areal percentage of the fibrotic region 35, and displays the evaluation information 71 on the display 40.

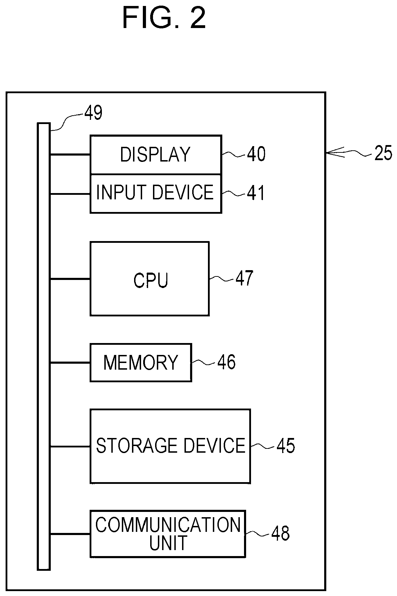

In Fig. 2, the computer constituting the pathological diagnosis support apparatus 25 includes a storage device 45, a memory 46, a central processing unit (CPU) 47, and a communication unit 48 in addition to the display 40 and the input device 41 described above. These are connected to each other via a busline 49.

The storage device 45 is a hard disk drive incorporated in the computer constituting the pathological diagnosis support apparatus 25 or connected thereto through a cable or a network. Alternatively, the storage device 45 is a disk array in which a plurality of hard disk drives are continuously mounted. The storage device 45 stores a control program such as an operating system, various application programs, various types of data accompanying these programs, and the like. A solid-state drive may be used instead of the hard disk drive.

The memory 46 is a work memory for the CPU 47 to execute processing. The CPU 47 loads a program stored in the storage device 45 to the memory 46 and performs processing in accordance with the program to generally control units of the computer.

The communication unit 48 is a network interface that controls transmission of various types of information via a network such as a local area network (LAN). The display 40 displays various screens. The computer constituting the pathological diagnosis support apparatus 25 receives an input of an operation instruction from the input device 41 through various screens.

In Fig. 3, an operation program 55 is stored in the storage device 45 of the pathological diagnosis support apparatus 25. The operation program 55 is an application program for causing the computer to function as the pathological diagnosis support apparatus 25. That is, the operation program 55 is an example of an "operation program of a pathological diagnosis support apparatus" according to the technology of the present disclosure. The storage device 45 also stores the sample image group 21G, a threshold value TH, and the like.

When the operation program 55 is started, the CPU 47 of the computer constituting the pathological diagnosis support apparatus 25 functions as a read/write (hereinafter abbreviated as RW) control unit 60, an instruction receiving unit 61, a detection unit 62, a derivation unit 63, and a display control unit 64 in cooperation with the memory 46 and the like. The CPU 47 is an example of a "processor" according to the technology of the present disclosure.

The RW control unit 60 controls storage of various types of data in the storage device 45 and reading out of various types of data in the storage device 45. For example, the RW control unit 60 receives the sample image group 21G transmitted from the imaging apparatus, and stores it in the storage device 45. In addition, the RW control unit 60 reads out, from the storage device 45, the sample image group 21G for which the instruction receiving unit 61 has received an image analysis instruction by a user via the input device 41, and outputs it to the detection unit 62, the derivation unit 63, and the display control unit 64. That is, the RW control unit 60 performs an "acquisition process" according to the technology of the present disclosure. The image analysis instruction by the user via the input device 41 is issued by inputting and designating, for example, the patient ID and the imaging date and time attached to the sample image 21.

Furthermore, the RW control unit 60 reads out the threshold value TH from the storage device 45, and outputs it to the detection unit 62.

The instruction receiving unit 61 receives various instructions from the user via the input device 41. The various instructions include the above-described image analysis instruction.

Based on the threshold value TH, the detection unit 62 detects the fibrotic region 35 from each of the plurality of sample images 21 constituting the sample image group 21G. That is, the detection unit 62 performs a "detection process" according to the technology of the present disclosure. The detection unit 62 outputs a detection result 70 of the fibrotic region 35 to the derivation unit 63 and the display control unit 64.

The derivation unit 63 derives the evaluation information 71 indicating the degree of fibrosis of the liver LV based on the detection result 70. That is, the derivation unit 63 performs a "derivation process" according to the technology of the present disclosure. The derivation unit 63 outputs the evaluation information 71 to the display control unit 64.

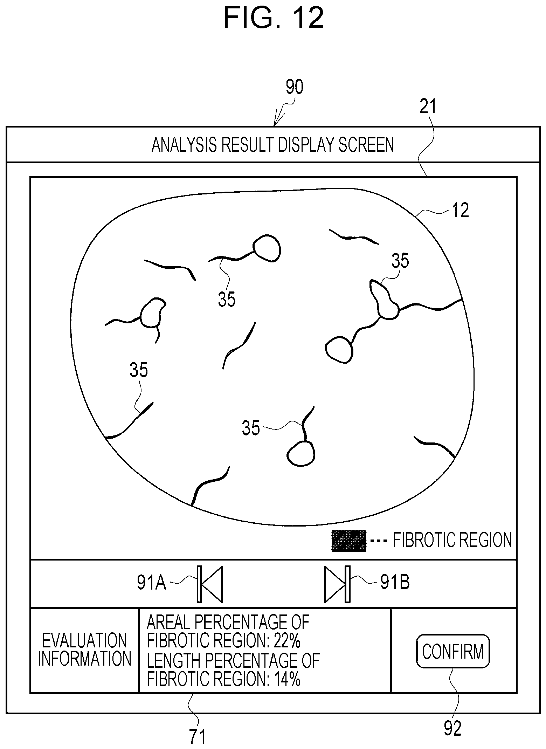

The display control unit 64 performs control to display various screens on the display 40. The various screens include an analysis result display screen 90 (see Fig. 12) representing a result of the image analysis. The display control unit 64 generates the analysis result display screen 90 based on the sample image group 21G, the detection result 70, and the evaluation information 71.

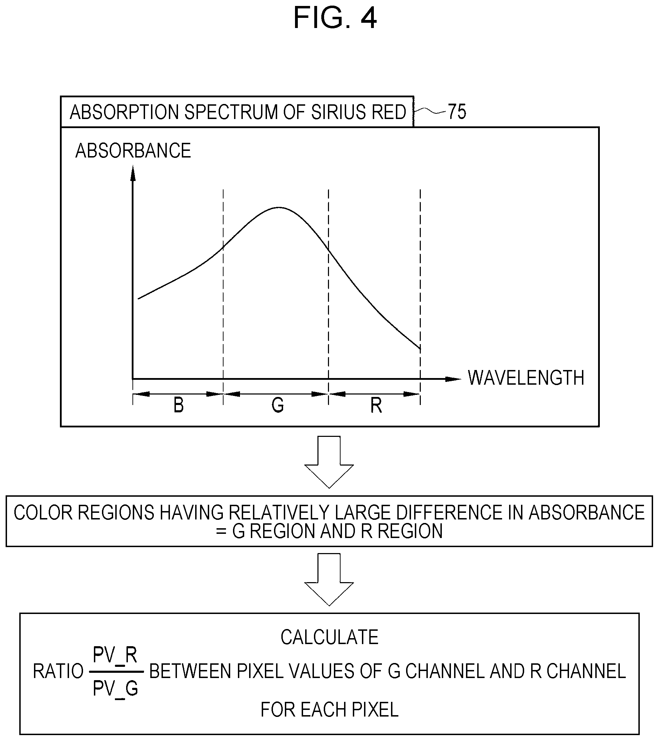

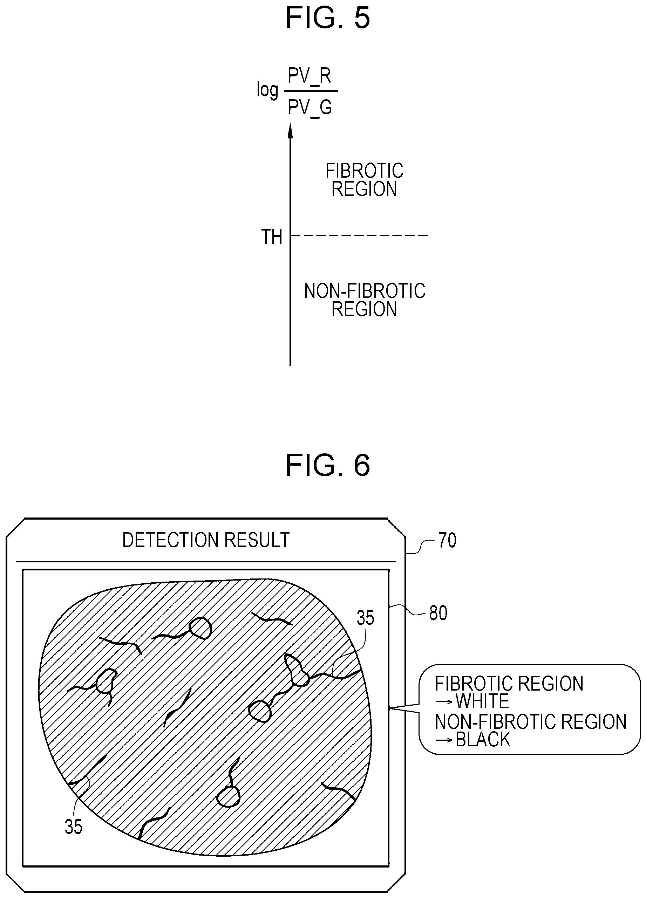

In Fig. 4, in an absorption spectrum 75 of Sirius red, the absorbance is highest in the G region, and then decreases in the order of the B region and the R region. Therefore, in the absorption spectrum 75 of Sirius red, the difference in absorbance is relatively larger between the G region and the R region than between the B region and the G region and between the B region and the R region. Therefore, the detection unit 62 calculates a ratio PV_R/PV_G between a pixel value PV_G of the G channel corresponding to the G region and a pixel value PV_R of the R channel corresponding to the R region for each pixel 30 of the sample image 21. The detection unit 62 log-transforms the ratio PV_R/PV_G to log(PV _R/PV_G) in order to obtain a numerical value that can be easily handled by a computer (see Fig. 5). Instead of the ratio PV_R/PV_G, a ratio PV_G/PV_R in which the denominator and the numerator are reversed may be calculated.

As illustrated in Fig. 5, the detection unit 62 compares log(PV_R/PV_G) with the threshold value TH. The detection unit 62 detects a pixel 30 in which log(PV_R/PV_G) is greater than or equal to the threshold value TH as the fibrotic region 35. On the other hand, the detection unit 62 detects a pixel 30 in which log(PV _R/PV_G) is less than the threshold value TH as a non-fibrotic region. The threshold value TH is a value empirically obtained from past knowledge.

As illustrated in Fig. 6, the detection unit 62 outputs a binarized image 80 as the detection result 70. The binarized image 80 is an image in which pixels 30 detected as the fibrotic region 35 are replaced with white (pixel value of each color channel = 255) and pixels 30 detected as the non-fibrotic region are replaced with black (pixel value of each color channel = 0).

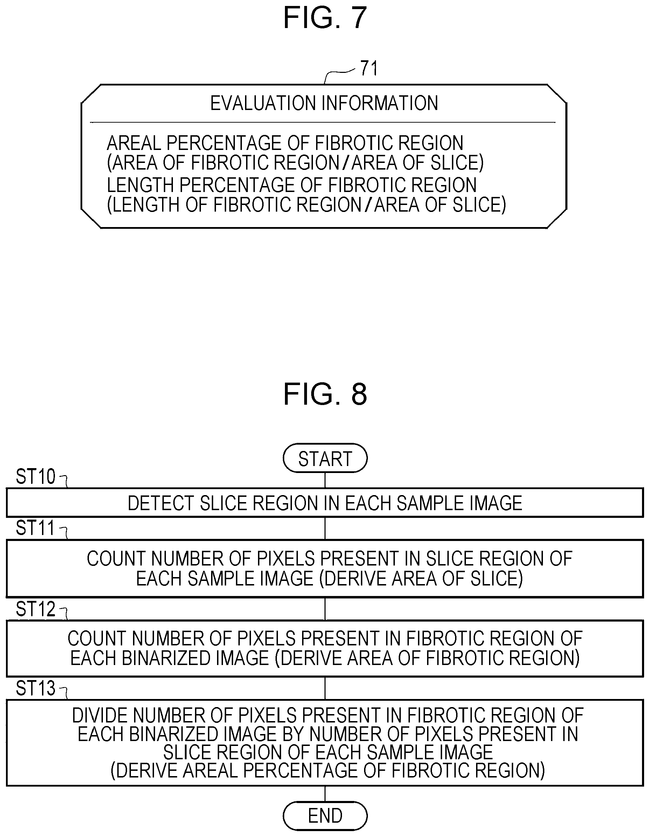

As illustrated in Fig. 7, the derivation unit 63 derives a numerical value related to at least one of the area or the length of the fibrotic region 35 as the evaluation information 71. More specifically, the evaluation information 71 includes the areal percentage of the fibrotic region 35 and the length percentage of the fibrotic region 35. The areal percentage of the fibrotic region 35 is a value obtained by dividing the area of the fibrotic region 35 by the area of the slice 12. The length percentage of the fibrotic region 35 is a value obtained by dividing the length of the fibrotic region 35 by the area of the slice 12.

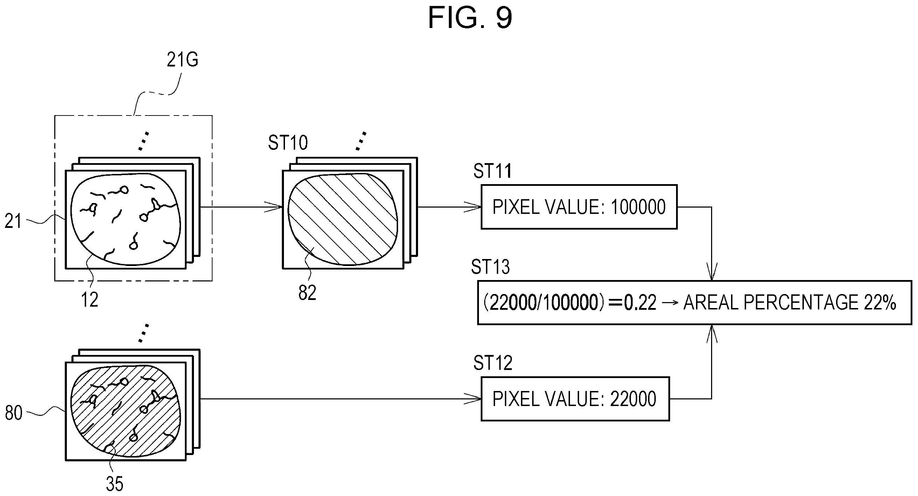

Figs. 8 and 9 illustrate a procedure for deriving the areal percentage of the fibrotic region 35 in the derivation unit 63. First, the derivation unit 63 detects a region (hereinafter referred to as a slice region) 82 of the slice 12 in each of the plurality of sample images 21 constituting the sample image group 21G (step ST10). For example, the derivation unit 63 determines a portion where a difference between pixel values of two adjacent pixels 30 is greater than or equal to a threshold value as a boundary between the slice 12 and the glass 13 outside the slice 12, and detects an inside of the boundary as the slice region 82.

Subsequently, the derivation unit 63 counts the number of pixels 30 present in the slice region 82 of each sample image 21 to derive the area of the slice 12 captured in each sample image 21 (step ST11). In addition, the derivation unit 63 counts the number of pixels 30 present in the fibrotic region 35 of each binarized image 80 corresponding to each sample image 21 to derive the area of the fibrotic region 35 (step ST12).

Finally, the derivation unit 63 divides the number of pixels 30 present in the fibrotic region 35 of each binarized image 80 counted in step ST12 by the number of pixels 30 present in the slice region 82 of each sample image 21 counted in step ST11 to derive the areal percentage of the fibrotic region 35 (step ST13).

Fig. 9 illustrates, as an example, a case where the number of pixels 30 present in the slice region 82 of each sample image 21 is 100000, and the number of pixels 30 present in the fibrotic region 35 of each binarized image 80 is 22000. In this case, the areal percentage of the fibrotic region 35 is (22000/100000) × 100 = 22%.

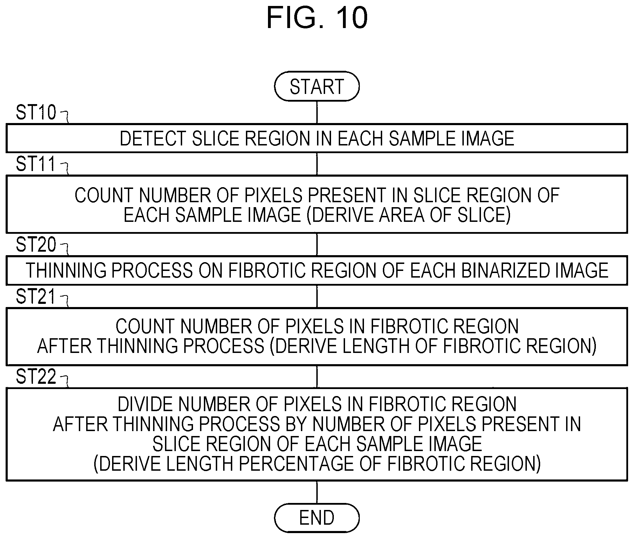

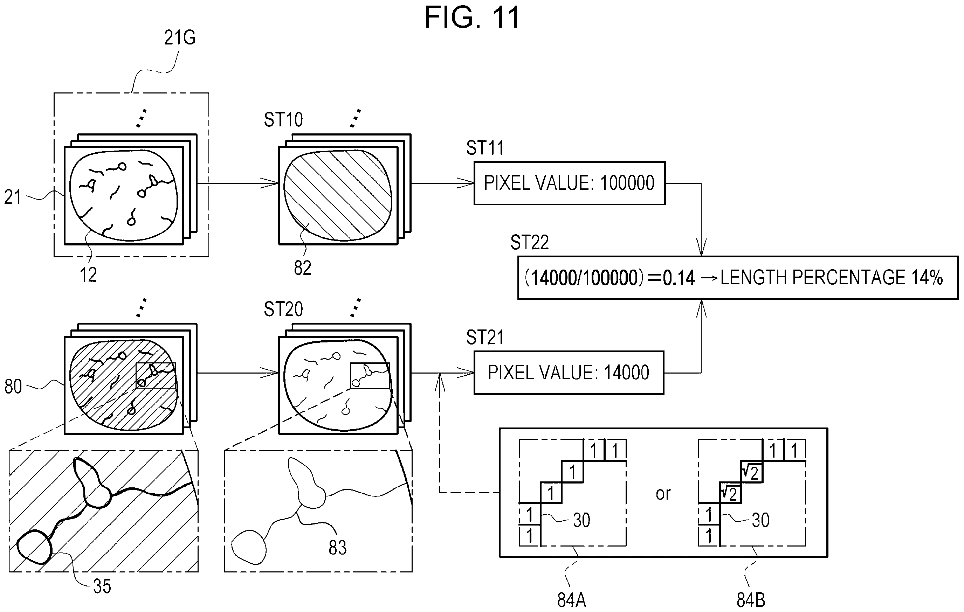

Figs. 10 and 11 illustrate a procedure for deriving the length percentage of the fibrotic region 35 in the derivation unit 63. The procedure up to step ST10 and step ST11 is the same as the procedure for deriving the areal percentage of the fibrotic region 35 illustrated in Figs. 8 and 9. After deriving the area of the slice 12 captured in each sample image 21 in step ST11, the derivation unit 63 performs a thinning process on the fibrotic region 35 of each binarized image 80 corresponding to each sample image 21 (step ST20). The thinning process is a process of converting the fibrotic region 35 into a line 83 having a width of one pixel and passing through the center of the width of the fibrotic region 35.

Subsequently, the derivation unit 63 counts the number of pixels 30 in the fibrotic region 35 after the thinning process, that is, the number of pixels 30 constituting the line 83, to derive the length of the fibrotic region 35 (step ST21). At this time, the derivation unit 63 counts the numbers of all pixels of the pixels 30 connected vertically, horizontally, and diagonally as 1, as illustrated in a two dot chain line frame 84A. Alternatively, the derivation unit 63 counts, among the pixels 30 constituting the line 83, the number of pixels 30 connected vertically and horizontally as 1, and the number of pixels 30 connected diagonally as √2, as illustrated in a two dot chain line frame 84B. As the length of the fibrotic region 35, the method illustrated in the frame 84B is more accurate.

Finally, the derivation unit 63 divides the number of pixels 30 in the fibrotic region 35 after the thinning process counted in step ST21 by the number of pixels 30 present in the slice region 82 of each sample image 21 counted in step ST11 to derive the length percentage of the fibrotic region 35 (step ST22).

Fig. 11 illustrates, as an example, a case where the number of pixels 30 present in the slice region 82 of each sample image 21 is 100000, as in the case in Fig. 9, and the number of pixels 30 in the fibrotic region 35 after the thinning process is 14000. In this case, the length percentage of the fibrotic region 35 is (14000/100000) × 100 = 14%.

Fig. 12 illustrates the analysis result display screen 90 displayed on the display 40 under the control of the display control unit 64. On the analysis result display screen 90, the sample image 21 is displayed in which the fibrotic region 35 detected by the detection unit 62 is painted in blue, for example. The sample image 21 displayed can be switched by operating a frame return button 91A and a frame advance button 91B.

The evaluation information 71 is displayed in the lower part of the analysis result display screen 90. That is, the display control unit 64 performs an "output process" according to the technology of the present disclosure.

A confirmation button 92 is provided next to the evaluation information 71. When the confirmation button 92 is selected, the display control unit 64 turns off the display of the analysis result display screen 90. The sample image 21 in which the fibrotic region 35 is distinguished in a specific color and the evaluation information 71 can be stored in the storage device 45 in response to an instruction from the user.

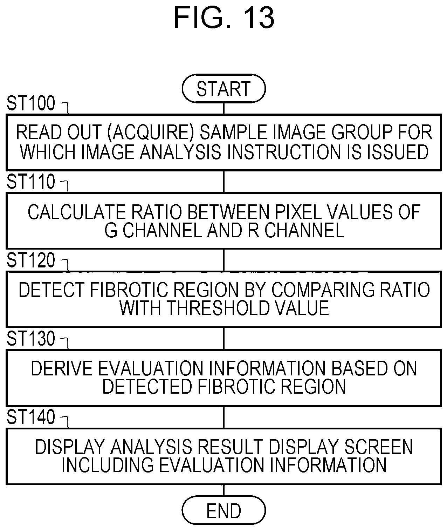

Next, operations of the above configuration will be described with reference to the flowchart in Fig. 13. First, when the operation program 55 is started in the pathological diagnosis support apparatus 25, as illustrated in Fig. 3, the CPU 47 of the pathological diagnosis support apparatus 25 functions as the RW control unit 60, the instruction receiving unit 61, the detection unit 62, the derivation unit 63, and the display control unit 64.

The RW control unit 60 reads out, from the storage device 45, the sample image group 21G for which the user has issued an image analysis instruction (step ST100). The sample image group 21G is output from the RW control unit 60 to the detection unit 62, the derivation unit 63, and the display control unit 64. Furthermore, the RW control unit 60 reads out the threshold value TH from the storage device 45, and outputs it to the detection unit 62. Step ST100 is an example of an "acquisition process" according to the technology of the present disclosure.

As illustrated in Fig. 4, the detection unit 62 calculates the ratio PV_R/PV_G between the pixel value PV_G of the G channel and the pixel value PV_R of the R channel for each pixel 30 of the sample image 21 (step ST110). Subsequently, as illustrated in Fig. 5, the logarithm log(PV_R/PV_G) of the ratio PV_R/PV_G is compared with the threshold value TH. Then, the pixels 30 in which log(PV _R/PV_G) is greater than or equal to the threshold value TH are detected as the fibrotic region 35, and the pixels 30 in which log (PV_R/PV_G) is less than the threshold value TH are detected as the non-fibrotic region (step ST120). The detection unit 62 generates the binarized image 80 illustrated in Fig. 6 as the detection result 70 of the fibrotic region 35. The detection unit 62 outputs the detection result 70 to the derivation unit 63 and the display control unit 64. Step ST120 is an example of a "detection process" according to the technology of the present disclosure.

As illustrated in Figs. 7 to 11, the derivation unit 63 derives the areal percentage of the fibrotic region 35 and the length percentage of the fibrotic region 35 as the evaluation information 71 (step ST130). The evaluation information 71 is output from the derivation unit 63 to the display control unit 64. Step ST130 is an example of a "derivation process" according to the technology of the present disclosure.

Under the control of the display control unit 64, the analysis result display screen 90 illustrated in Fig. 12 is displayed on the display 40 (step ST140). Accordingly, the fibrotic region 35 and the evaluation information 71 are presented to the user. The user grasps the degree of fibrosis in the liver LV of the patient P who is a target of pathological diagnosis on the analysis result display screen 90. Step ST140 is an example of an "output process" according to the technology of the present disclosure.

As described above, the pathological diagnosis support apparatus 25 includes the RW control unit 60, the detection unit 62, the derivation unit 63, and the display control unit 64. The RW control unit 60 acquires the sample image 21 obtained by imaging the sample 20 of the liver LV stained with Sirius red by reading it out from the storage device 45. The detection unit 62 detects the fibrotic region 35 of the liver LV by comparing, with the preset threshold value TH, the ratio PV_R/PV_G between the pixel values PV_G and PV_R of the G channel and the R channel among the three color channels of RGB of the sample image 21. The derivation unit 63 derives the evaluation information 71 indicating the degree of fibrosis of the liver LV based on the detected fibrotic region 35. The display control unit 64 outputs the evaluation information 71 by displaying the analysis result display screen 90 including the evaluation information 71 on the display 40.

The ratio PV_R/PV_G is more robust than the pixel value PV_G or PV_R itself with respect to changes in the color of staining due to differences in the concentration of Sirius red, the ambient environment, the imaging apparatus of the sample image 21, and the like. This can improve the detection accuracy of the fibrotic region 35. Therefore, the fibrotic region 35, which has been detected intermittently due to low detection accuracy of the fibrotic region 35 in spite of actual bridging fibrosis, can be reliably recognized as bridging fibrosis.

In the absorption spectrum 75 of Sirius red, the detection unit 62 calculates the ratio PV_R/PV_G between the pixel values PV_G and PV_R of the G channel and the R channel corresponding to the G region and the R region in which the difference in absorbance is relatively large. Therefore, it is possible to widen the dynamic range of the ratio and further improve the detection accuracy of the fibrotic region 35.

The derivation unit 63 derives, as the evaluation information 71, a numerical value related to at least one of the area or the length of the fibrotic region 35, specifically, the areal percentage of the fibrotic region 35 and the length percentage of the fibrotic region 35. Therefore, the degree of fibrosis of the liver LV can be objectively evaluated. When the numerical value related to the length of the fibrotic region 35 is relatively large, it can be estimated that fibrosis has progressed in a relatively wide range of the liver LV.

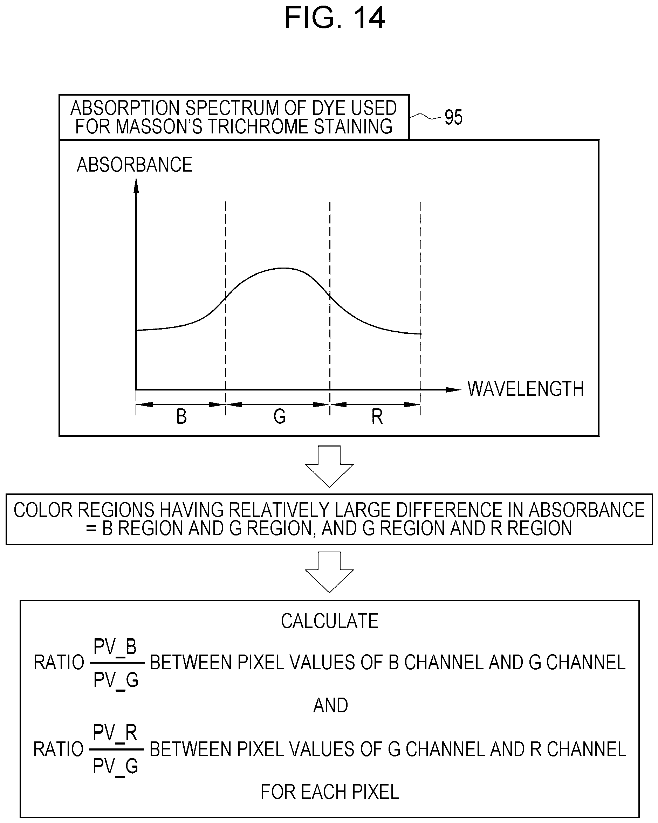

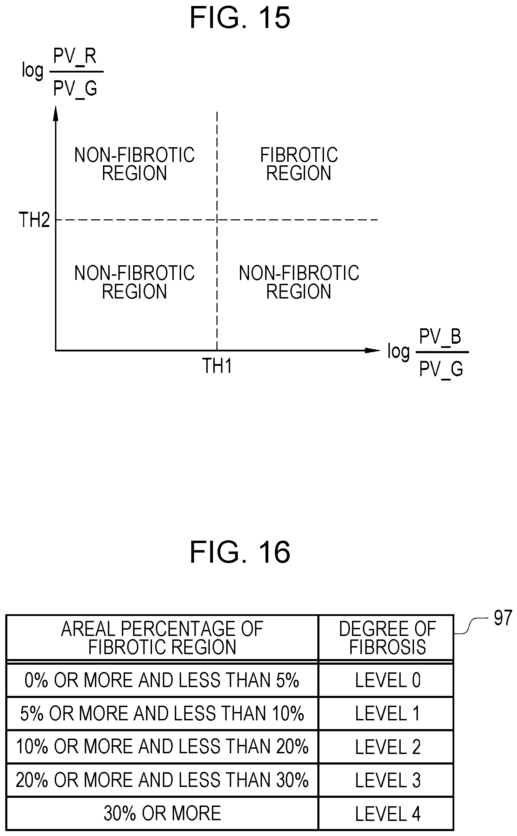

The dye is not limited to Sirius red illustrated as an example. For example, iron hematoxylin, Ponceau xylidine, acid fuchsin, and aniline blue used for Masson's trichrome staining may be used. In a case of the dye used for Masson's trichrome staining, as illustrated in an absorption spectrum 95 in Fig. 14, the absorbance in the G region is relatively high, and the absorbances in the B region and the R region are relatively low. In addition, there is almost no difference in absorbance between the B region and the R region. Therefore, in the absorption spectrum 95 of the dye used for Masson's trichrome staining, the difference in absorbance is relatively larger between the B region and the G region and the G region and the R region than between the B region and the R region. Therefore, in this case, the detection unit 62 calculates a ratio PV_B/PV_G between the pixel value PV_B of the B channel corresponding to the B region and the pixel value PV_G of the G channel corresponding to the G region, and the ratio PV_R/PV_G between the pixel value PV_G of the G channel corresponding to the G region and the pixel value PV_R of the R channel corresponding to the R region, for each pixel 30 of the sample image 21. The detection unit 62 log-transforms the ratio PV_B/PV_G to log(PV_B/PV_G) and log-transforms the ratio PV_R/PV_G to log (PV_R/PV_G) (see Fig. 15). A ratio PV_G/PV_B and a ratio PV_G/PV_R may be calculated.

As illustrated in Fig. 15, the detection unit 62 compares log(PV_B/PV_G) with a first threshold value TH1. In addition, the detection unit 62 compares log(PV _R/PV_G) with a second threshold value TH2. The detection unit 62 detects a pixel 30 in which log(PV _B/PV_G) is greater than or equal to the first threshold value TH1 and log(PV _R/PV_G) is greater than or equal to the second threshold value TH2 as the fibrotic region 35. On the other hand, the detection unit 62 detects a pixel 30 in which log(PV _B/PV_G) is less than the first threshold value TH1 or log(PV _R/PV_G) is less than the second threshold value TH2 as the non-fibrotic region. Like the threshold value TH, the first threshold value TH1 and the second threshold value TH2 are values empirically obtained from past knowledge.

As described above, the ratio between the pixel values of the two color channels calculated for detecting the fibrotic region 35 is not limited to one type. Note that a pixel 30 in which log(PV_B/PV_G) is less than the first threshold value TH1 and log (PV_R/PV_G) is less than the second threshold value TH2 may be detected as the non-fibrotic region, and a pixel 30 in which log (PV _B/PV_G) is greater than or equal to the first threshold value TH1 or log (PV _R/PV_G) is greater than or equal to the second threshold value TH2 may be detected as the fibrotic region 35.

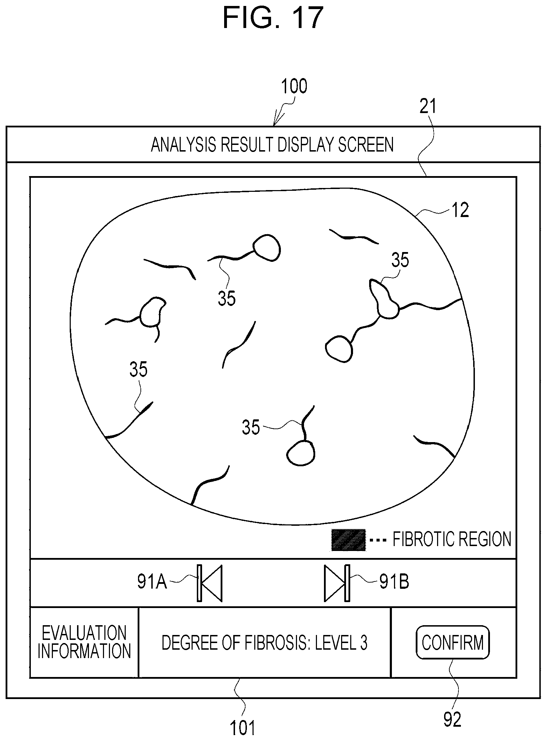

The evaluation information 71 is not limited to the areal percentage of the fibrotic region 35 and the length percentage of the fibrotic region 35 illustrated as examples above. For example, the areal percentage of the fibrotic region 35 may be changed to five levels representing the degrees of fibrosis with reference to a table 97 illustrated in Fig. 16, and the levels may be derived as evaluation information 101 (see Fig. 17). In the table 97, levels corresponding to ranges of the areal percentage of the fibrotic region 35 are registered. For example, level 0 is registered when the areal percentage of the fibrotic region 35 is 0% or more and less than 5%, level 3 is registered when the areal percentage is 20% or more and less than 30%, and level 4 is registered when the areal percentage is 30% or more. The table 97 is stored in the storage device 45, and is read out by the RW control unit 60 and output to the derivation unit 63.

Fig. 17 illustrates an analysis result display screen 100 on which a level indicating the degree of fibrosis is displayed as the evaluation information 101.

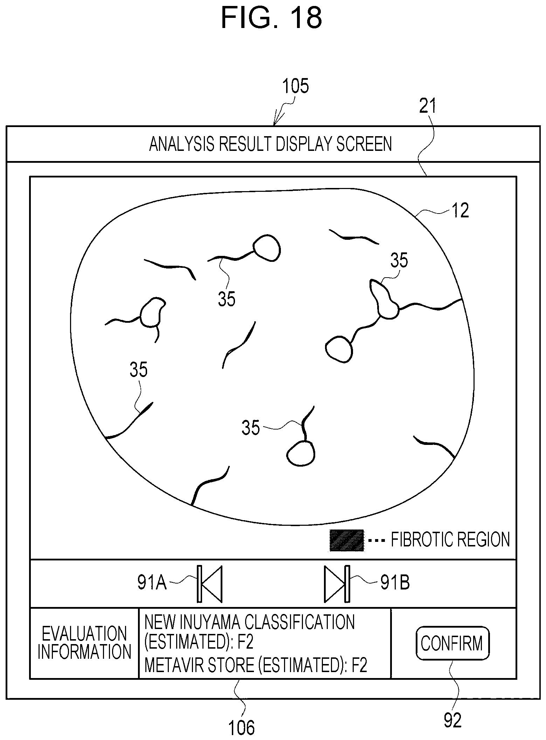

In addition, as in an analysis result display screen 105 illustrated in Fig. 18, stages of known fibrosis evaluation indexes such as new Inuyama classification and METAVIR score may be derived and output as evaluation information 106. Each stage of the new Inuyama classification and the METAVIR score is derived from an estimation expression using, for example, the areal percentage of the fibrotic region 35 and the length percentage of the fibrotic region 35 as parameters and using the stage as a solution. In addition, non-alcoholic fatty liver disease (NAFLD) activity score (NAS) or the like may be derived and output as evaluation information.

### Second Embodiment

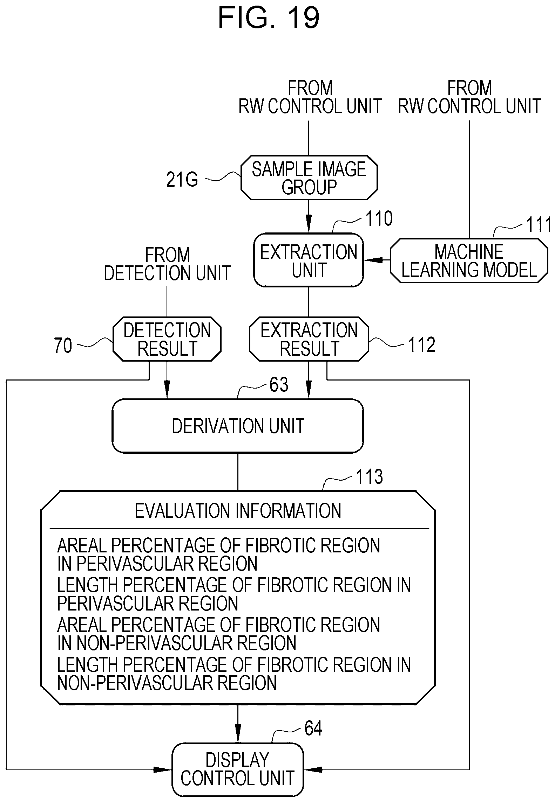

In a second embodiment illustrated in Figs. 19 to 22, evaluation information 113 is derived separately for the perivascular region 36 and a non-perivascular region 115 other than the perivascular region 36.

As illustrated in Fig. 19, a CPU of a pathological diagnosis support apparatus according to the second embodiment functions as an extraction unit 110 in addition to the units 60 to 64 (omitted from the illustration in Fig. 19 except the derivation unit 63 and the display control unit 64) according to the first embodiment above. The extraction unit 110 extracts the perivascular region 36 and the non-perivascular region 115 from each of the sample images 21 constituting the sample image group 21G using a machine learning model 111.

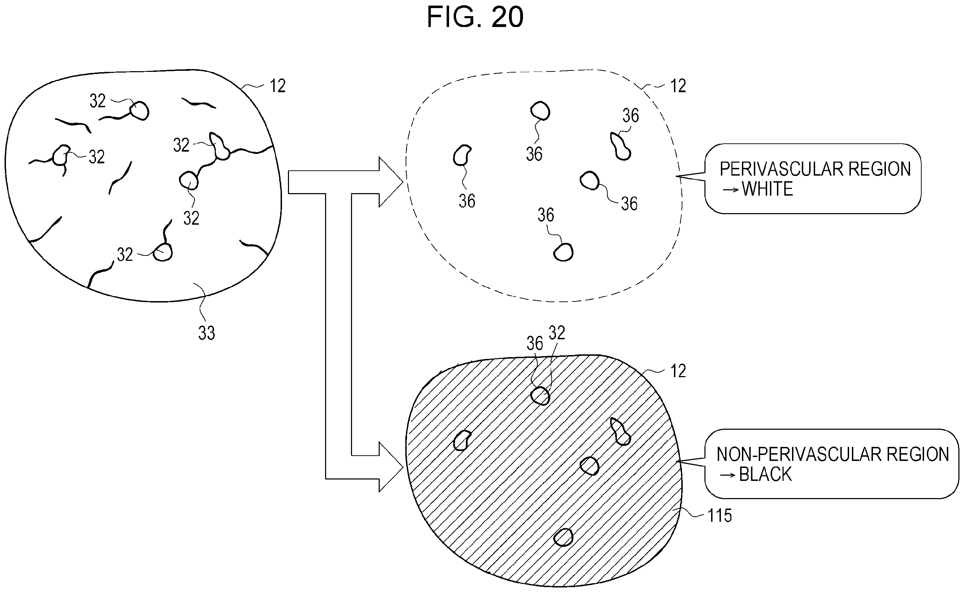

The machine learning model 111 is, for example, a convolutional neural network such as U-shaped neural network (U-Net), SegNet, residual network (ResNet), or densely connected convolutional network (DenseNet). The machine learning model 111 is stored in the storage device 45, and is read out by the RW control unit 60 and output to the extraction unit 110. The machine learning model 111 is a model in which the sample image 21 is used as an input image, and a binarized image in which the pixels 30 in the perivascular region 36 are replaced with white and the pixels 30 in the non-perivascular region 115 are replaced with black is used as an output image as illustrated in Fig. 20, for example. The machine learning model 111 extracts, for example, a region having a width of about 20 pixels surrounding the periphery of the vascular region 32 as the perivascular region 36. In addition, the machine learning model 111 extracts the pixels 30 of the slice 12 other than the pixels 30 extracted as the perivascular region 36 as the non-perivascular region 115. Thus, the non-perivascular region 115 includes the vascular region 32 and most of the parenchymal region 33. The machine learning model 111 is trained using a plurality of sets of a sample image 21 for learning and a correct image in which the perivascular region 36 of the sample image 21 for learning is designated by a user's hand.

In Fig. 19, the extraction unit 110 outputs the binarized image in which the pixels 30 in the perivascular region 36 are replaced with white and the pixels 30 in the non-perivascular region 115 are replaced with black to the derivation unit 63 and the display control unit 64 as an extraction result 112. Based on the extraction result 112 from the extraction unit 110 and the detection result 70 from the detection unit 62, the derivation unit 63 derives, as the evaluation information 113, the areal percentage of the fibrotic region 35 in the perivascular region 36 and the length percentage of the fibrotic region 35 in the perivascular region 36. In addition, the derivation unit 63 derives, as the evaluation information 113, the areal percentage of the fibrotic region 35 in the non-perivascular region 115 and the length percentage of the fibrotic region 35 in the non-perivascular region 115.

The areal percentage of the fibrotic region 35 in the non-perivascular region 36 is derived in step ST12 illustrated in Figs. 8 and 9 by counting the number of pixels 30 present in the fibrotic region 35 in the non-perivascular region 36 and then dividing this by the number of pixels 30 present in the slice region 82 of each sample image 21 counted in step ST11. The length percentage of the fibrotic region 35 in the non-perivascular region 36 is derived in step ST21 illustrated in Figs. 10 and 11 by counting the number of pixels 30 in the fibrotic region 35 in the non-perivascular region 36 after the thinning process and then dividing this by the number of pixels 30 present in the slice region 82 of each sample image 21 counted in step ST11. The same applies to the areal percentage of the fibrotic region 35 in the non-perivascular region 115 and the length percentage of the fibrotic region 35 in the non-perivascular region 115.

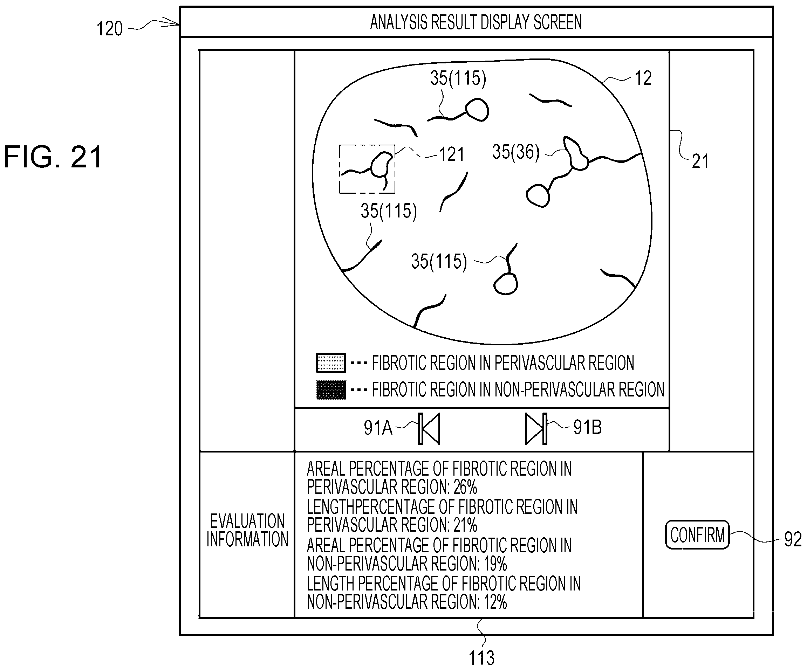

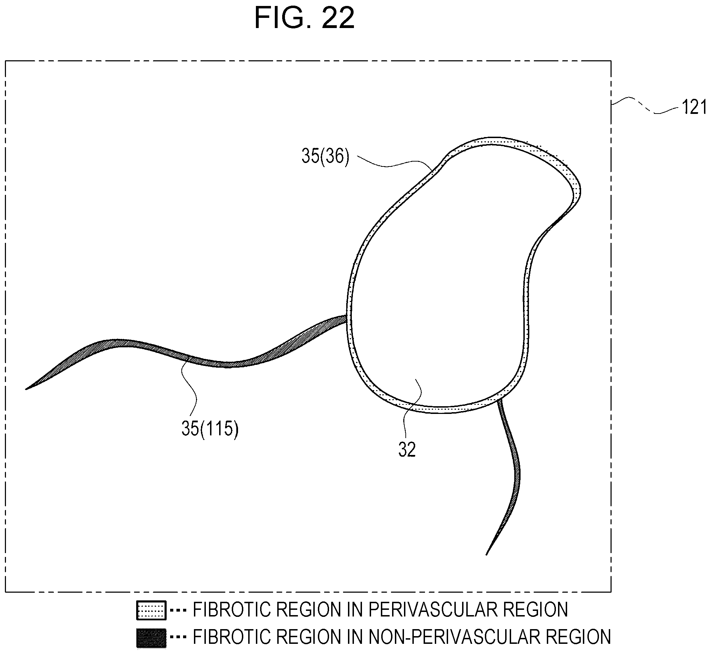

The display control unit 64 displays an analysis result display screen 120 illustrated in Fig. 21 on the display 40. On the analysis result display screen 120, as also illustrated in Fig. 22 which is an enlarged view of a frame 121, the sample image 21 is displayed in which the fibrotic region 35 in the perivascular region 36 is painted in green, for example, and the fibrotic region 35 in the non-perivascular region 115 is painted in blue, for example. In addition, as the evaluation information 113, the areal percentage and the length percentage of the fibrotic region 35 in the perivascular region 36 and the areal percentage and the length percentage of the fibrotic region 35 in the non-perivascular region 115 are displayed.

In this manner, in the second embodiment, the evaluation information 71 is derived separately for the perivascular region 36 and the non-perivascular region 115. Therefore, it is possible to evaluate a disease for which the progression of fibrosis is mainly observed in the perivascular region 36 and a disease for which the progression of fibrosis is mainly observed in the non-perivascular region 115 while distinguishing the disease states from each other.

In clinical trials using rats or the like, a fatty liver is intentionally caused by injecting a drug such as carbon tetrachloride. Therefore, in any case, fibrosis is likely to progress in the perivascular region 36 due to the influence of the drug. Therefore, when the evaluation information 71 is derived separately for the perivascular region 36 and the non-perivascular region 115 as in the second embodiment, it is possible to evaluate the degree of fibrosis excluding the influence of the drug.

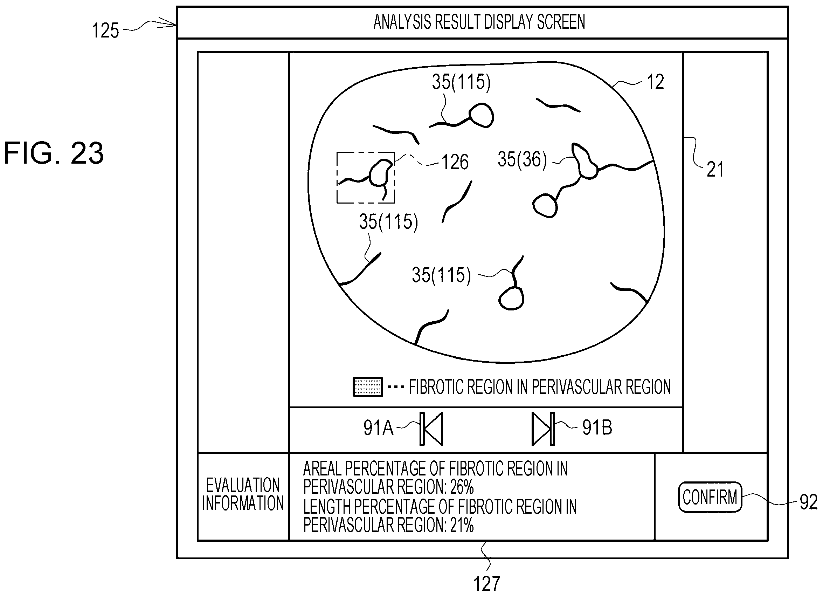

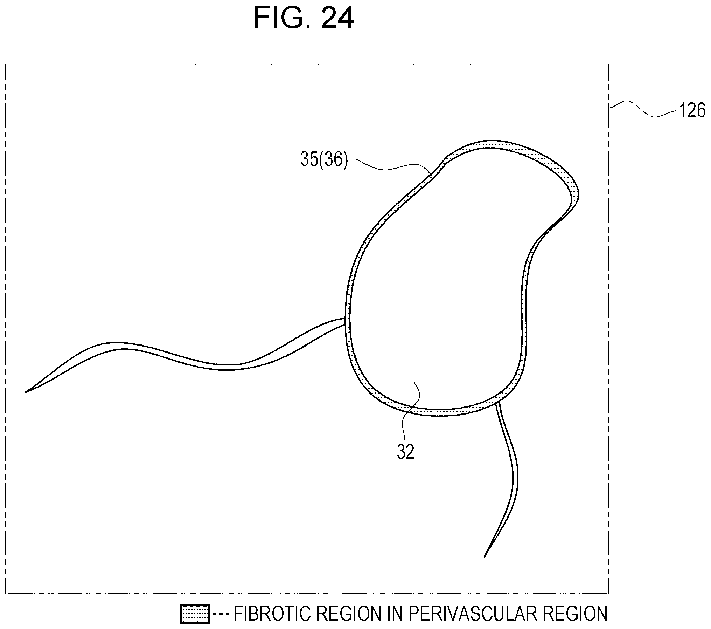

In the analysis result display screen 120 illustrated in Fig. 21, the colors of the fibrotic region 35 in the perivascular region 36 and the fibrotic region 35 in the non-perivascular region 115 are different, and the areal percentage and the length percentage of the fibrotic region 35 in the perivascular region 36 and the areal percentage and the length percentage of the fibrotic region 35 in the non-perivascular region 115 are displayed as the evaluation information 113. However, the present disclosure is not limited to this. As in an analysis result display screen 125 illustrated in Fig. 23, only the fibrotic region 35 in the perivascular region 36 may be painted in green, for example (see also Fig. 24 which is an enlarged view of a frame 126), and only the areal percentage and the length percentage of the fibrotic region 35 in the perivascular region 36 may be displayed as the evaluation information 127. Alternatively, although illustration is omitted, in contrast to the case of Fig. 23, only the fibrotic region 35 in the non-perivascular region 115 may be painted in blue, for example, and only the areal percentage and the length percentage of the fibrotic region 35 in the non-perivascular region 115 may be displayed as the evaluation information. The display mode illustrated in Fig. 21, the display mode illustrated in Fig. 23, and the display mode opposite to the case of Fig. 23 may be configured to be switchable.

The perivascular region 36 may be extracted without using the machine learning model 111. For example, the vascular region 32 is extracted using a known image recognition technique, and a region surrounding the periphery of the extracted vascular region 32 and having a width of 20 pixels, for example, is extracted as the perivascular region 36. Alternatively, the user may be caused to designate the perivascular region 36.

### Third Embodiment

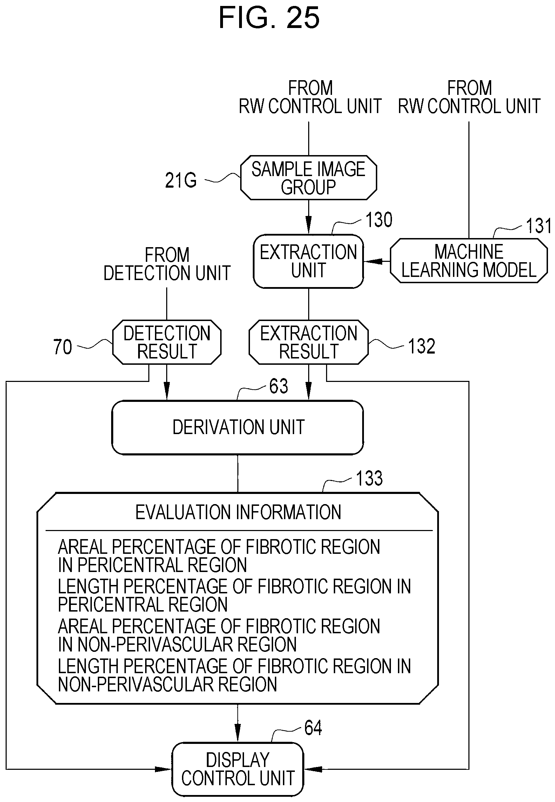

In a third embodiment illustrated in Figs. 25 to 28, evaluation information 133 is derived separately for a pericentral region 36C and the non-perivascular region 115. The pericentral region 36C includes the periphery of the central vein passing through the liver LV.

As illustrated in Fig. 25, an extraction unit 130 according to the third embodiment extracts the pericentral region 36C, a periportal region 36P, and the non-perivascular region 115 from each of the sample images 21 constituting the sample image group 21G using a machine learning model 131.

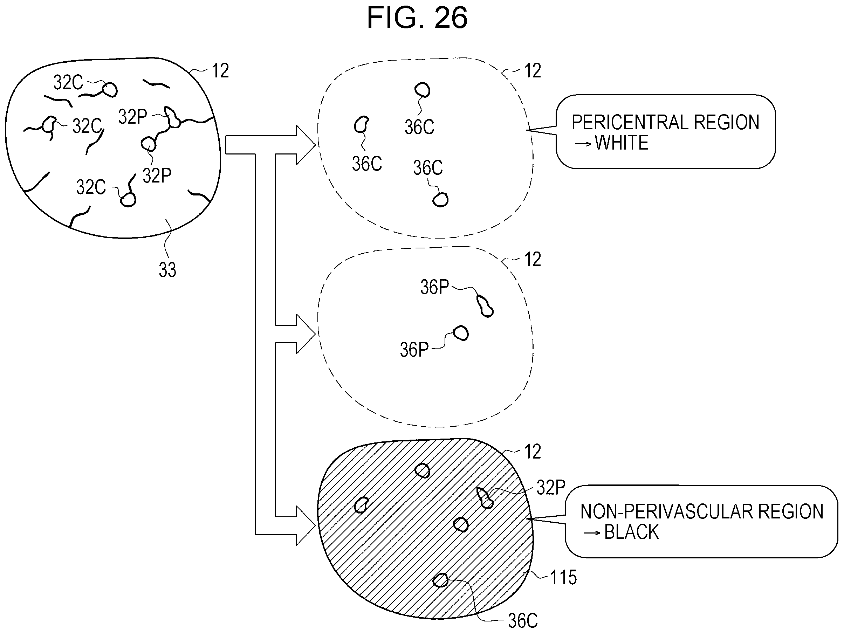

The machine learning model 131 is a convolutional neural network such as U-Net, similarly to the machine learning model 111 according to the second embodiment above. The machine learning model 131 is stored in the storage device 45, and is read out by the RW control unit 60 and output to the extraction unit 130. The machine learning model 131 is a model in which the sample image 21 is used as an input image, and a binarized image in which the pixels 30 in the pericentral region 36C are replaced with white and the pixels 30 in the non-perivascular region 115 are replaced with black is used as an output image as illustrated in Fig. 26, for example. The machine learning model 131 extracts, for example, a region having a width of about 20 pixels surrounding the periphery of a central vein region 32C as the pericentral region 36C. Similarly, the machine learning model 131 extracts, for example, a region having a width of about 20 pixels surrounding the periphery of a portal vein region 32P as the periportal region 36C. In addition, the machine learning model 131 extracts the pixels 30 of the slice 12 other than the pixels 30 extracted as the pericentral region 36C and the periportal region 36P as the non-perivascular region 115. The machine learning model 131 is trained using a plurality of sets of a sample image 21 for learning and a correct image in which the pericentral region 36C and the periportal region 36P of the sample image 21 for learning are designated by a user's hand.

In Fig. 25, the extraction unit 130 outputs the binarized image in which the pixels 30 in the pericentral region 36C are replaced with white and the pixels 30 in the non-perivascular region 115 are replaced with black to the derivation unit 63 and the display control unit 64 as an extraction result 132. Based on the extraction result 132 from the extraction unit 130 and the detection result 70 from the detection unit 62, the derivation unit 63 derives, as the evaluation information 133, the areal percentage of the fibrotic region 35 in the pericentral region 36C and the length percentage of the fibrotic region 35 in the pericentral region 36C. In addition, the derivation unit 63 derives, as the evaluation information 133, the areal percentage of the fibrotic region 35 in the non-perivascular region 115 and the length percentage of the fibrotic region 35 in the non-perivascular region 115.

The areal percentage of the fibrotic region 35 in the pericentral region 36C is derived in step ST12 illustrated in Figs. 8 and 9 by counting the number of pixels 30 present in the fibrotic region 35 in the pericentral region 36C and then dividing this by the number of pixels 30 present in the slice region 82 of each sample image 21 counted in step ST11. The length percentage of the fibrotic region 35 in the pericentral region 36C is derived in step ST21 illustrated in Figs. 10 and 11 by counting the number of pixels 30 in the fibrotic region 35 in the perivascular region 36 after the thinning process and then dividing this by the number of pixels 30 present in the slice region 82 of each sample image 21 counted in step ST11.

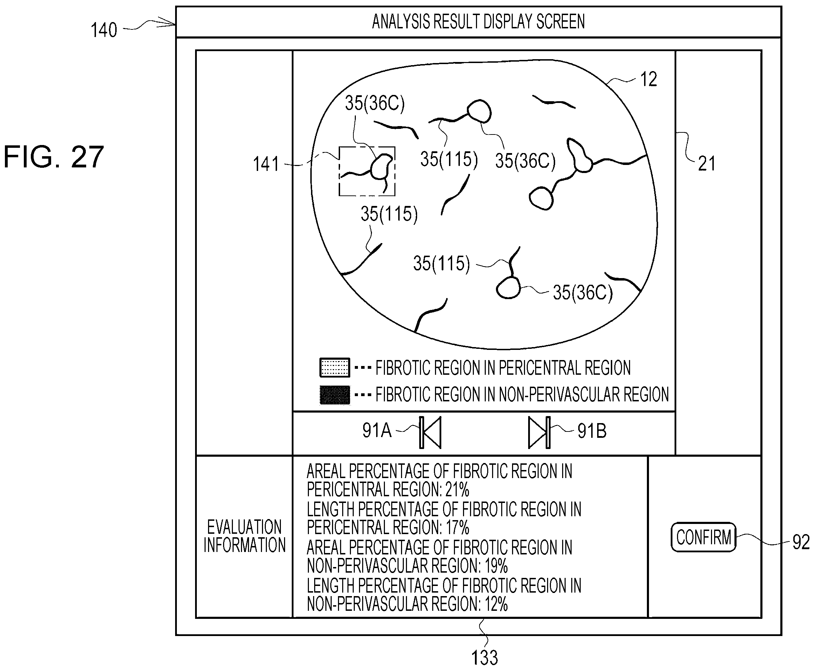

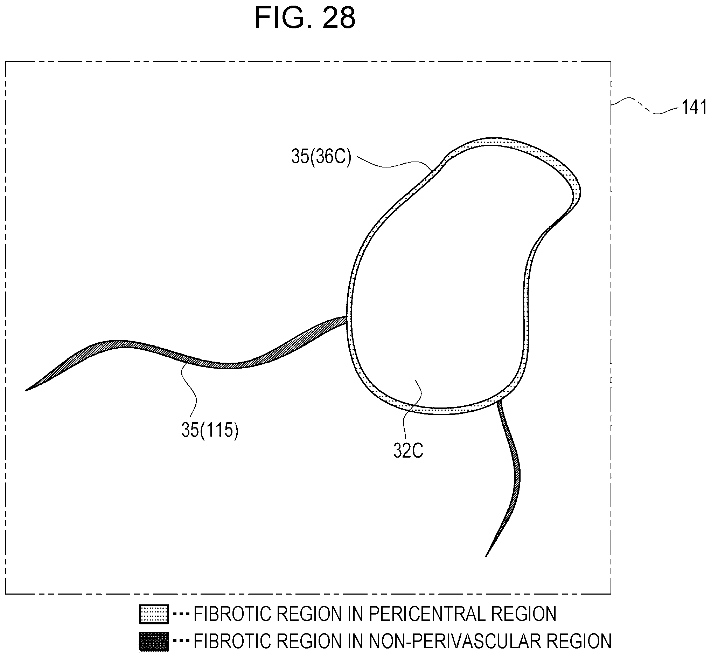

The display control unit 64 displays an analysis result display screen 140 illustrated in Fig. 27 on the display 40. On the analysis result display screen 140, as also illustrated in Fig. 28 which is an enlarged view of a frame 141, the sample image 21 is displayed in which the fibrotic region 35 in the pericentral region 36C is painted in green, for example, and the fibrotic region 35 in the non-perivascular region 115 is painted in blue, for example. In addition, as the evaluation information 133, the areal percentage and the length percentage of the fibrotic region 35 in the pericentral region 36C and the areal percentage and the length percentage of the fibrotic region 35 in the non-perivascular region 115 are displayed.

In this manner, in the third embodiment, the evaluation information 71 is derived separately for the pericentral region 36C and the non-perivascular region 115. Therefore, it is possible to correctly evaluate the state of a disease such as non-alcoholic steatohepatitis (NASH) in which fibrosis progresses from the pericentral region 36C toward the non-perivascular region 115.

As in the second embodiment above, only the fibrotic region 35 in the pericentral region 36C may be painted in green, for example, and only the areal percentage and the length percentage of the fibrotic region 35 in the pericentral region 36C may be displayed as the evaluation information. This display mode and the display mode illustrated in Fig. 27 may be configured to be switchable. Alternatively, the pericentral region 36C may be extracted without using the machine learning model 111 by, for example, causing the user to designate the pericentral region 36C.

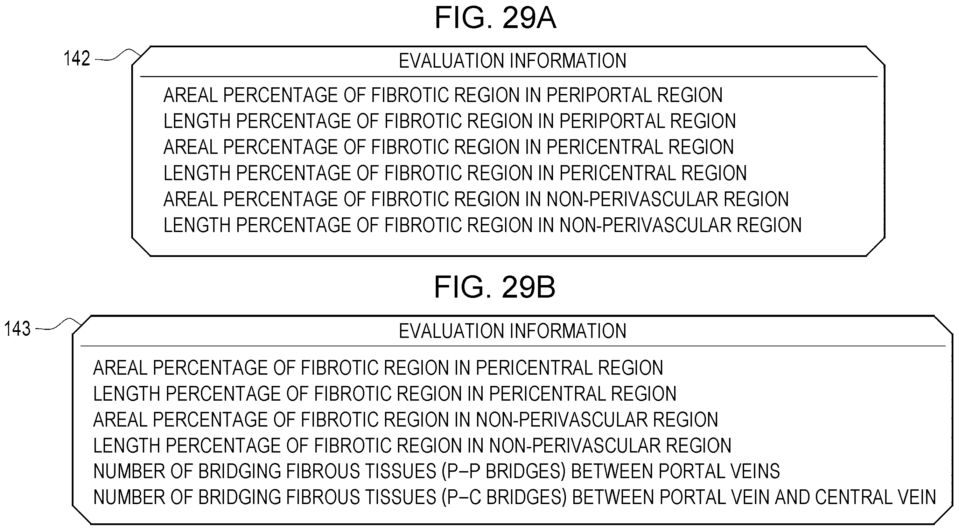

As in evaluation information 142 illustrated in Fig. 29A, not only the areal percentage and the length percentage of the fibrotic region 35 in the pericentral region 36C, but also the areal percentage and the length percentage of the fibrotic region 35 in the periportal region 36P may be derived. In addition, as in evaluation information 143 illustrated in Fig. 29B, bridging fibrosis between portal veins, that is, the number of P-P bridges, and bridging fibrosis between a portal vein and a central vein, that is, the number of P-C bridges may be derived.

### Fourth Embodiment

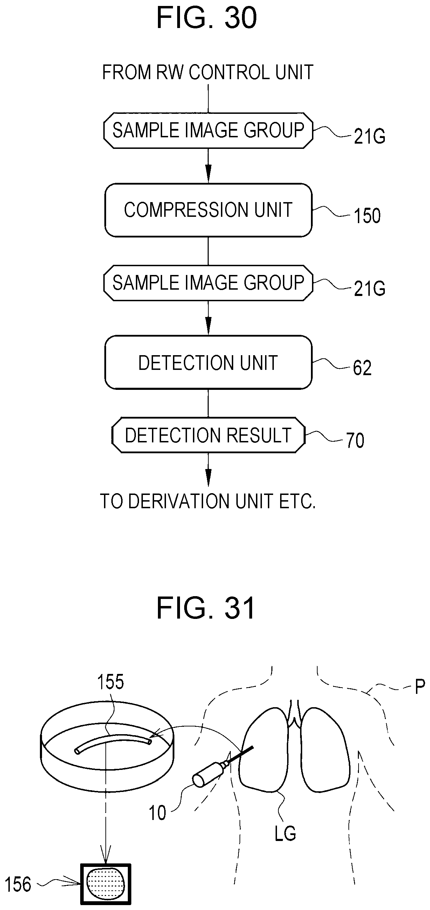

In a fourth embodiment illustrated in Fig. 30, after the sample images 21 are subjected to compression processing, the fibrotic region 35 is detected.

In Fig. 30, a CPU of a pathological diagnosis support apparatus according to the fourth embodiment functions as a compression unit 150 in addition to the units 60 to 64 (omitted from the illustration in Fig. 30 except the detection unit 62) according to the first embodiment above. The sample image group 21G read out from the storage device 45 by the RW control unit 60 is input to the compression unit 150. The compression unit 150 performs compression processing on each of the plurality of sample images 21 constituting the sample image group 21G. The compression unit 150 outputs the sample image group 21G after the compression processing to the detection unit 62. The detection unit 62 detects the fibrotic region 35 from the compressed sample image 21, and outputs the detection result 70 to the derivation unit 63 and the like.

As described above, in the fourth embodiment, after the compression processing is performed by the compression unit 150 on the sample images 21 acquired by the RW control unit 60, the detection unit 62 detects the fibrotic region 35. Therefore, it is possible to reduce a processing load as compared with a case where the fibrotic region 35 is detected from the full-size sample images 21.

The above embodiments have illustrated the sample 20 collected from the liver LV of the patient P as an example. However, the present disclosure is not limited this. For example, as illustrated in Fig. 31, a sample 156 may be prepared from a test substance 155 collected from a lung LG of the patient P using the biopsy needle 10. It may also be a sample collected from skin, breast, stomach, large intestine, and the like.

The color channels of the pixel values of the sample image 21 are not limited to the illustrated RGB color channels. They may be four color channels of CMYG (Cyan, Magenta, Yellow, and Green).

The output form of the evaluation information is not limited to the form of displaying on the analysis result display screen. The evaluation information may be printed out on a paper medium, or a data file of the evaluation information may be transmitted and output by e-mail or the like.

Various modifications can be made to the hardware configuration of the computer constituting the pathological diagnosis support apparatus. The pathological diagnosis support apparatus may be configured by a plurality of computers separated as hardware for the purpose of improving processing capability and reliability. For example, the functions of the RW control unit 60 and the instruction receiving unit 61 and the functions of the detection unit 62, the derivation unit 63, and the display control unit 64 are distributed to two computers. In this case, two computers constitute the pathological diagnosis support apparatus.

As described above, the hardware configuration of the computer of the pathological diagnosis support apparatus can be changed as appropriate in accordance with required performance such as processing capability, safety, and reliability. Furthermore, not only hardware but also application programs such as the operation program 55 can be duplicated or distributed and stored in a plurality of storage devices for the purpose of ensuring safety and reliability.

In each of the above-described embodiments, for example, as a hardware structure of a processing unit that executes various kinds of processing such as the RW control unit 60, the instruction receiving unit 61, the detection unit 62, the derivation unit 63, the display control unit 64, the extraction units 110 and 130, and the compression unit 150, various processors described below can be used. Various processors include, in addition to the CPU 47, which is a general-purpose processor functioning as various processing units by executing software (the operation program 55), a programmable logic device (PLD) that is a processor in which the circuit configuration is changeable after manufacture, such as field programmable gate array (FPGA), a dedicated electric circuit that is a processor having a circuit configuration that is specially designed to execute specific processing, such as an application specific integrated circuit (ASIC), and the like.

One processing unit may be constituted by one of these various processors, or may be constituted by two or more processors of the same type or different types in combination (e.g., a combination of a plurality of FPGAs, and/or a combination of a CPU and an FPGA). In addition, a plurality of processing units may be configured as one processor.

As a first example for constituting a plurality of processing units with one processor, one processor may be constituted by a combination of one or more CPUs and software, and this processor may function as a plurality of processing units, as typified by a computer such as a client or a server. As a second example, a processor may be used that implements the functions of the entire system including a plurality of processing units as one integrated circuit (IC) chip, as typified by a system on chip (SoC) or the like. In this manner, various processing units are constituted by one or more of the above various processors in terms of hardware configuration.

Furthermore, the hardware configuration of these various processors may be electric circuitry constituted by combining circuit elements such as semiconductor elements.

In the technology of the present disclosure, the above-described various embodiments and various modifications may be combined as appropriate. The present disclosure is not limited to the above-described embodiments, and various configurations can be employed without departing from the gist of the present disclosure, as a matter of course. Furthermore, the technology of the present disclosure covers not only the program but also a recording medium having the program stored therein in a non-transitory manner.

The content described above and illustrated in the drawings is a detailed description of portions related to the technology of the present disclosure, and is merely an example of the technology of the present disclosure. For example, the above description regarding the configuration, the function, the operation, and the effect is a description regarding an example of the configuration, the function, the operation, and the effect of the portions related to the technology of the present disclosure. Therefore, it is needless to say that unnecessary portions may be deleted, new elements may be added, or replacement may be performed with respect to the content described above and illustrated in the drawings without departing from the gist of the technology of the present disclosure. In addition, in order to avoid complexity and to facilitate understanding of portions related to the technology of the present disclosure, description of common technical knowledge and the like that do not particularly require description in order to enable implementation of the technology of the present disclosure is omitted from the content described above and illustrated in the drawings.

As used herein, "A and/or B" is synonymous with "at least one of A or B". That is, "A and/or B" means that A may be employed alone, B may be employed alone, or A and B may be employed in combination. In addition, in the present specification, when three or more matters are expressed by being combined with "and/or", the same concept as "A and/or B" is applied.

All documents, patent applications, and technical standards mentioned in the present specification are incorporated herein by reference to the same extent as if individual document, patent application, or technical standard is specifically and individually indicated to be incorporated by reference.

## Claims

1. A pathological diagnosis support apparatus comprising at least one processor configured to:
acquire a sample image obtained by imaging a sample of a biological tissue stained with a dye, the sample image having pixel values of a plurality of color channels;
detect a fibrotic region of the biological tissue by comparing a ratio between pixel values of two color channels among the plurality of color channels with a preset threshold value;
derive evaluation information indicating a degree of fibrosis of the biological tissue based on the detected fibrotic region; and
output the evaluation information.

2. The pathological diagnosis support apparatus according to claim 1, wherein the processor calculates a ratio between pixel values of two color channels corresponding to two color regions having a relatively large difference in absorbance in an absorption spectrum of the dye.

3. The pathological diagnosis support apparatus according to claim 1, wherein the processor derives the evaluation information separately for a perivascular region and a non-perivascular region other than the perivascular region, the perivascular region including a periphery of a blood vessel passing through the biological tissue.

4. The pathological diagnosis support apparatus according to claim 3, wherein
the sample is collected from a liver, and
the processor derives the evaluation information separately for a pericentral region and the non-perivascular region, the pericentral region including a periphery of a central vein passing through the liver.

5. The pathological diagnosis support apparatus according to any one of claims 1 to 4, wherein the processor derives a numerical value related to at least any one of an area or a length of the fibrotic region as the evaluation information.

6. The pathological diagnosis support apparatus according to any one of claims 1 to 5, wherein the processor detects the fibrotic region after performing compression processing on the acquired sample image.

7. An operation method for a pathological diagnosis support apparatus, wherein a processor executes:
an acquisition process of acquiring a sample image obtained by imaging a sample of a biological tissue stained with a dye, the sample image having pixel values of a plurality of color channels;
a detection process of detecting a fibrotic region of the biological tissue by comparing a ratio between pixel values of two color channels among the plurality of color channels with a preset threshold value;
a derivation process of deriving evaluation information indicating a degree of fibrosis of the biological tissue based on the detected fibrotic region; and
an output process of outputting the evaluation information.

8. An operation program for a pathological diagnosis support apparatus, the program causing a processor to execute:
an acquisition process of acquiring a sample image obtained by imaging a sample of a biological tissue stained with a dye, the sample image having pixel values of a plurality of color channels;
a detection process of detecting a fibrotic region of the biological tissue by comparing a ratio between pixel values of two color channels among the plurality of color channels with a preset threshold value;
a derivation process of deriving evaluation information indicating a degree of fibrosis of the biological tissue based on the detected fibrotic region; and
an output process of outputting the evaluation information.
